# EUROPEAN PATENT APPLICATION

(11) **EP 4 722 334 A1**
(43) Date of publication of application: **08.04.2026**
(21) Application number: 23939795.3
(22) Date of filing: 20.12.2023
(51) Int. Cl.: C12M 1/04, C12M 3/00

(54) **AUTOMATIC CULTURE DEVICE AND AUTOMATIC CULTURE SYSTEM**

(30) Priority: 02.06.2023 JP 2023091700
(71) Applicant: HITACHI HIGH-TECH CORPORATION, Tokyo 105-6409 (JP)
(72) Inventor: SAITO Hikaru, Tokyo 100-8280 (JP); KIYAMA Masaharu, Tokyo 100-8280 (JP); KOBAYASHI Toyoshige, Tokyo 100-8280 (JP)
(74) Representative: MERH-IP Matias Erny Reichl Hoffmann Patentanwälte PartG mbB
(86) International application number: PCT/JP2023/045673
(87) International publication number: WO 2024/247328

(57) **Abstract**

In order to provide an automatic culture device and an automatic culture system in which a device housing can be miniaturized and a plurality of devices can be simultaneously operated in a space-saving manner, the following configuration was adopted.

An automatic culture system includes: an automatic culture device capable of accommodating a culture vessel; and a centralized management computer for controlling a plurality of automatic culture devices. The automatic culture device includes a temperature maintaining mechanism configured to maintain the culture vessel at a predetermined temperature, a gas supply unit configured to supply humidified CO₂ gas, and a ventilation adapter attached to the culture vessel and configured to supply the humidified CO₂ gas from the gas supply unit to the culture vessel.

## Description

### Technical Field

The present invention relates to an automatic culture device for culturing cells or tissues, and more particularly, to an automatic culture device that can be miniaturized and an automatic culture system using a plurality of automatic culture devices.

### Background Art

A medical treatment using regenerated human cells and cells with modified functions through gene transfer can treat diseases that have been difficult to cure so far. Therefore, the medical treatment is expected to be widely used.

The most common treatment target is a cancer, followed by various organs. This is considered to be because autogenous transplantations using the cells of patients have a low possibility of rejection and are in high demand from the perspective of improving patient quality of life (QOL).

The most common cell type is immune cells, accounting for more than 40% of the total. The main target disease is cancer, and specific cell types include T cells, NK cells, NKT cells, and the like. In particular, the practical application of chimeric antigen receptor-T (CAR-T) cell therapy, in which T cells, which are immune cells, are extracted from a patient, processed by gene transfer into a form capable of attacking cancer cells, and then returned to the patient by injection, is currently at the forefront.

In a step of producing cells for transplantation, a biological sample taken from a patient or another person is separated and purified, and subjected to processing such as amplification and genetic introduction.

This step is performed in a cell processing center (CPC) in accordance with a standard operating procedure (SOP) satisfying a good manufacturing practice (GMP) that is a standard for manufacturing management and quality control of pharmaceutical products and the like. Therefore, the operation of the CPC requires significant costs and personnel with specialized culture techniques.

In addition, since the manufacturing step is mainly performed manually, there is a limit to an increase in the manufacturing amount. The low productivity and the high manufacturing costs are impeding the widespread adoption of regenerative medicine, and there is a demand for automation of culture work that particularly requires labor and costs in a manufacturing step. It is possible to save labor, reduce costs, and achieve mass production due to automation of the culture work.

Examples of an automatic culture device include a device in which a closed flow path having a closed space is automatically handled as shown in PTL 1. In the closed flow path, a closed culture vessel is always connected to a flow path tube or the like, and cells are cultured inside the closed culture vessel.

The closed flow path enables the movement of a liquid and a gas held therein by an operation of a valve, a pump, and the like disposed outside the closed flow path. Accordingly, the automatic culture device automatically performs cell seeding, culture medium change, microscopic observation, and the like while maintaining the closing property of the culture space.

### Citation List

### Patent Literature

PTL 1: JP2007-312668A

### Summary of Invention

### Technical Problem

In the technique described in PTL 1, culture can be performed in a closed culture vessel. However, in order to perform the culture, the culture vessel is held in a CO₂ incubator whose inside is maintained at a constant CO₂ gas concentration. Gas exchange is performed by dissolving a gas in a space present above the culture medium in the culture vessel into the culture medium or releasing a gas in the culture medium into the space.

However, the CO₂ incubator is large from the viewpoint of ensuring airtightness for filling the internal space with gas, and therefore, the automatic culture device using the configuration is hardly miniaturized.

In addition, the inside of the CO₂ incubator is humidified, and therefore, it is difficult to mount mechanical elements such as a swing mechanism for a culture vessel used for a culture operation and a camera observation mechanism for cells because a metal component is in an environment where rust is easily generated.

In addition, the CO₂ incubator has a risk of generating microorganisms due to humidification, and there is an operational hurdle for use in a production facility of cells for transplantation requiring high cleanliness.

An object of the invention is to provide an automatic culture device in which the size of the device can be reduced, and a plurality of devices can be simultaneously operated in a space-saving manner, and an automatic culture system using the plurality of automatic culture devices.

### Solution to Problem

A configuration of the invention for achieving the above object is as follows.

An automatic culture device capable of accommodating a culture vessel includes a temperature maintaining mechanism configured to maintain the culture vessel at a predetermined temperature, a gas supply unit configured to supply humidified CO₂ gas, and a ventilation adapter attached to the culture vessel and configured to supply the humidified CO₂ gas from the gas supply unit to the culture vessel.

### Advantageous Effects of Invention

The invention can provide an automatic culture device in which the size of the device can be reduced, and a plurality of devices can be simultaneously operated in a space-saving manner, and an automatic culture system using the plurality of automatic culture devices.

### Brief Description of Drawings

[FIG. 1] FIG. 1 is a view illustrating a configuration of an automatic culture device according to Embodiment 1.
[FIG. 2] FIG. 2 is a side view illustrating a configuration of the automatic culture device according to Embodiment 1.
[FIG. 3A] FIG. 3A is a view illustrating an example of a ventilation adapter according to Embodiment 1.
[FIG. 3B] FIG. 3B is a view illustrating an example of the ventilation adapter according to Embodiment 1.
[FIG. 3C] FIG. 3C is a view illustrating an example of the ventilation adapter according to Embodiment 1.
[FIG. 4] FIG. 4 is a top view of the ventilation adapter in Embodiment 1.
[FIG. 5] FIG. 5 is a view illustrating a result of gas exchange in a culture vessel by the ventilation adapter in Embodiment 1.
[FIG. 6A] FIG. 6A is a view illustrating an example of a flow path circuit including a closed culture vessel according to Embodiment 1.
[FIG. 6B] FIG. 6B is a diagram illustrating an example of a procedure of cell seeding according to Embodiment 1.
[FIG. 6C] FIG. 6C is a diagram illustrating an example of a gas exchange procedure according to Embodiment 1.
[FIG. 6D] FIG. 6D is a diagram illustrating an example of a culture medium addition procedure according to Embodiment 1.
[FIG. 6E] FIG. 6E is a diagram illustrating an example of culture medium change procedure according to Embodiment 1.
[FIG. 6F] FIG. 6F is a diagram illustrating an example of a supernatant sampling procedure according to Embodiment 1.
[FIG. 6G] FIG. 6G is a diagram illustrating an example of a cell collection procedure according to Embodiment 1.
[FIG. 7] FIG. 7 is a diagram illustrating a state of inclination of the culture vessel during the cell collection according to Embodiment 1.
[FIG. 8] FIG. 8 is a diagram illustrating a flow during an operation of the automatic culture device according to Embodiment 1.
[FIG. 9] FIG. 9 is a diagram illustrating an example of the ventilation adapter for a flask culture vessel according to Embodiment 2.
[FIG. 10] FIG. 10 is a diagram illustrating an example of an automatic culture system in which a plurality of automatic culture devices according to Embodiment 1 are connected in parallel and controlled by a central management PC.

### Description of Embodiments

Hereinafter, embodiments of the invention will be described with reference to the drawings. The following description shows a specific example of the content of the invention, and the invention is not limited to the description, and various changes and modifications can be made by those skilled in the art within the scope of the technical idea disclosed in the description.

In addition, in all the drawings for illustrating the invention, components having the same functions are denoted by the same reference signs, and repeated description thereof may be omitted.

### [Embodiment 1]

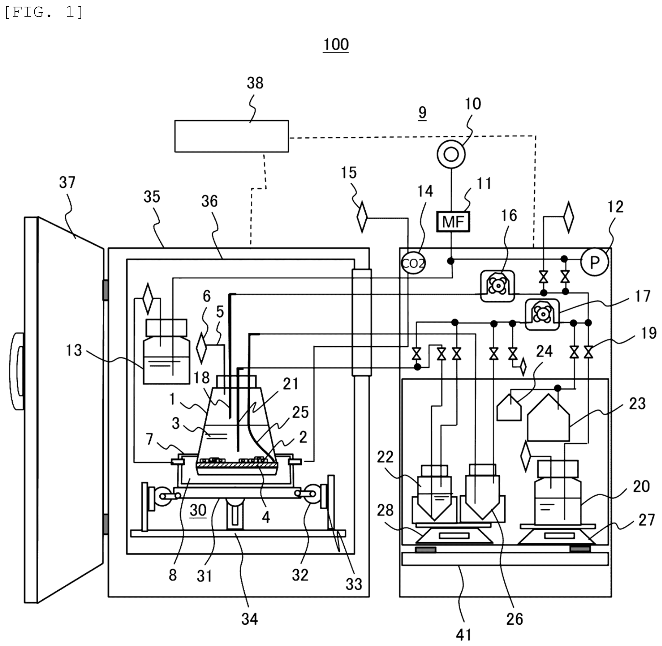

Components of an automatic culture device of the present embodiment, which performs culture using a closed culture vessel, will be described with reference to FIG. 1.

An automatic culture device 100 includes a culture vessel 1, a ventilation adapter 7, a gas supply unit 9, a pump for feeding a liquid or feeding a gas, flow paths for connecting the components, valves for opening and closing the flow paths, a control unit 38 for controlling the gas supply unit, the pump, the valves, and the like, a swing mechanism 30 for swinging the culture vessel 1, and an incubator 35 as a temperature maintaining mechanism for accommodating the culture vessel, the ventilation adapter, the swing mechanism, and the like and performing temperature control. The culture vessel 1 is a ventilation surface-equipped culture vessel having a ventilation surface 4 with a gas-permeable membrane disposed on a bottom surface, and cells 2 are held together with a culture medium 3 and cultured therein. A pressure regulating pipe 5 and a vent filter 6 are connected to the culture vessel 1, and thus a gas inside the culture vessel can flow in and out, and the entry of bacteria and viruses from the outside is prevented.

In addition, the culture vessel 1 is attached to the ventilation adapter 7, and gas supply and exchange necessary for culture can be performed in the culture vessel 1. The gas supply unit 9 includes a gas cylinder 10 in which a predetermined gas concentration is maintained, a gas flow rate control unit (mass flow controller: MF) 11, a pressure sensor 12, and a humidification bottle 13 serving as a humidification unit, and is connected upstream of the ventilation adapter 7. A CO₂ sensor 14 and a CO₂ gas vent filter 15 are connected downstream of the ventilation adapter 7, and the gas is discharged to the atmosphere outside the device. The CO₂ sensor 14 can monitor whether the gas exchange in the ventilation adapter is appropriately performed, and can be used to predict a culture state.

The feeding of a liquid or the feeding of a gas is performed by pumps 16 and 17, and a preferable pump is a tube pump that generates pressure by squeezing a rubber tube with a rotating roller.

The pump 16 is configured to feed a culture medium to the culture vessel 1 and is connected to a liquid feeding pipe 18 of the culture vessel 1 by a tube. On the other hand, the pump 16 is connected to a supply pipe of a culture medium bottle 20 via a solenoid valve 19.

A solenoid valve is preferable as a valve for opening and closing a rubber tube constituting the flow path, and when electricity is applied, the solenoid valve is operated to open the rubber tube in the closed state which is clamped by the valve operated by a spring force.

The pump 17 is configured to feed a cell suspension to the culture vessel 1 to perform cell seeding, or discharge the culture medium 3 from the culture vessel 1 to collect grown cells.

The pump 17 is connected to an aspirating tube 21 of the culture vessel 1 by a tube. On the other hand, the pump 17 is connected to a cell seeding bottle 22 via the solenoid valve 19 and is connected to a supernatant collection bag 23 and a supernatant analysis bag 24 via another solenoid valve 19.

The pump 17 is connected to a collection pipe 25 for cells in the culture vessel 1 by a tube, and on the other hand, is connected to a cell collection bottle 26 via the solenoid valve 19.

The weight of the culture medium bottle 20 is measured by a weight sensor 27, and the weight of the cell seeding bottle 22 and the weight of the cell collection bottle 26 are measured by a weight sensor 28.

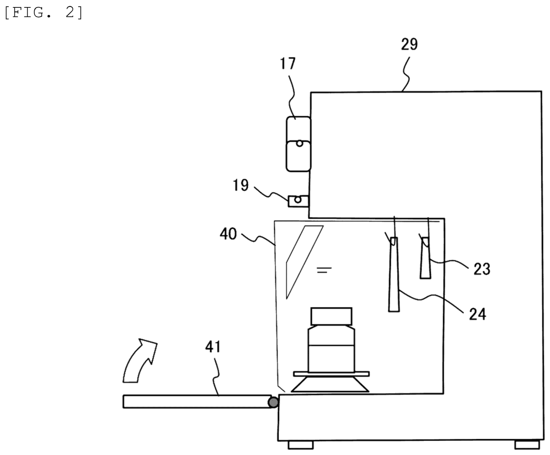

As illustrated in FIGS. 1 and 2, the culture medium bottle, the cell seeding bottle, the cell collection bottle, the supernatant collection bag, the supernatant analysis bag, the flow path connecting the components, the pump, the solenoid valve, and the weight sensor are provided in a fluid control unit 29 which is a device body outside the incubator 35.

The swing mechanism 30 includes a swing stage 31 for holding the ventilation adapter 7, link mechanisms 32 for supporting the swing stage from three directions, swing shafts 33 respectively connected to the link mechanisms 32, and a swing stage fixing mechanism 34 fixed to an inside of the incubator.

In a swing operation of the culture vessel, when control is performed such that the right swing shaft is lowered downward, the left swing shaft is raised upward simultaneously, and the central swing shaft is not moved, the swing stage moves with a tilt, and the culture vessel can be tilted. Next, when the motion of the left and right shafts is reversed, the culture vessel can be tilted in an opposite direction.

When the swing shafts are continuously operated and the swing shaft is also moved in a depth direction of the paper, the culture vessel can be tilted back and forth, and therefore, the cells 2 and the culture medium 3 inside the culture vessel 1 can be stirred.

The incubator 35 is an example of a temperature maintaining mechanism, and is a so-called dry incubator including a constant temperature unit 36 and an opening/closing door 37. The incubator 35 can house the culture vessel 1, the swing mechanism 30, and the humidification bottle 13, and can maintain the inside of the incubator at a temperature suitable for cell culture. By adopting a small-sized dry incubator instead of a large-sized CO₂ incubator and having a configuration capable of performing gas supply necessary for culture, the device can be downsized, and a plurality of devices can be simultaneously operated in a small space. The control unit 38 can control the operations of the gas supply unit 9, the pumps 16 and 17, and the solenoid valve 19, and the operation of the swing mechanism 30. By automatically controlling the mechanical elements at a predetermined timing, the cell suspension can be fed from the cell seeding bottle 22 to the culture vessel 1 during cell seeding, the gas humidified by the humidification bottle 13 can be supplied to the ventilation adapter 7 during gas exchange, the culture medium can be fed from the culture medium bottle 20 to the culture vessel 1 during culture medium addition, a culture medium can be supplied to the culture vessel 1 after the culture medium 3 in the culture vessel 1 is discharged to the supernatant collection bag 23 during culture medium change, and a part of the culture medium in the culture vessel can be fed to the supernatant analysis bag 24 during supernatant sampling.

During cell collection, after the culture medium in the culture vessel 1 is discharged to the supernatant collection bag 23, the cell suspension can be stirred by operating the swing mechanism 30 and then fed to the cell collection bottle 26.

FIG. 2 is a right side view of the device according to Embodiment 1. Side covers 40 are provided on the left and right sides of the device, respectively, and a bottle unit door 41 that can be opened and closed in the horizontal and vertical directions is provided on the front of the device.

The side cover 40 has the effect of a windshield for blocking the movement of the gas in the entire space in which the culture medium bottle 20 and the cell seeding bottle 22 and the cell collection bottle 26 are placed on the weight sensors 27 and 28 and measured, and performing stable measurement.

The bottle unit door 41 is in an open state when in a horizontal position, and has an effect of a placement table on which a bottle or the like is temporarily placed when a flow path including a bottle or a tube is placed. After the tube in the flow path is connected to the pump or the valve, the tube is closed in a vertical direction and can be used as a windshield. The opening and closing directions of the bottle unit door 41 and the opening/closing door 37 of the incubator 35 are different from each other, and therefore, both doors can be opened and closed without interference, and the device can be easily accessed during work.

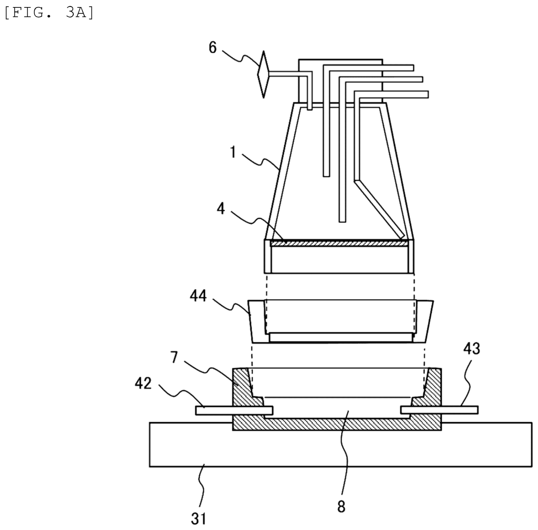

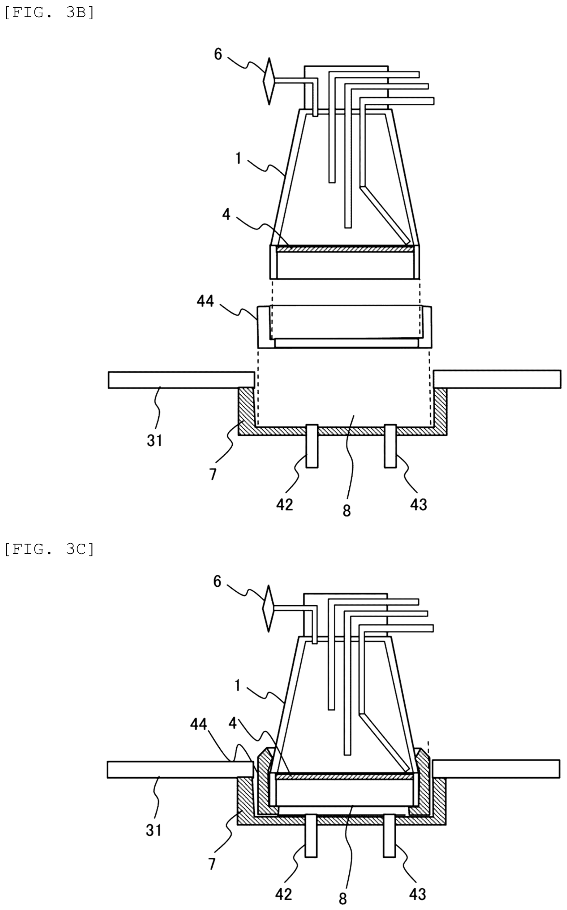

Components of the ventilation adapter 7 according to Embodiment 1 will be described with reference to FIGS. 3A, 3B, and 3C. In FIG. 3A, the ventilation adapter 7 is placed on the swing stage 31, and is connected to a gas feeding pipe 42 connected to the humidification bottle 13 and an exhaust pipe 43 connected to the CO₂ sensor. The culture vessel 1 is pressed against a rubber packing 44 mainly by the weight of the culture vessel 1 and the elasticity of the rubber packing 44 on a horizontal table (not shown), so that the culture vessel 1 is closely attached to the ventilation adapter 7. At this time, a predetermined gas space portion 8 is formed below the culture vessel 1 and inside the ventilation adapter 7.

FIG. 3A illustrates an example of a ventilation adapter that supplies CO₂ gas from the outside of the circumference of the ventilation adapter 7 and exhausts the gas from the outside of the circumference. The ventilation adapter of this type is characterized in that the height direction can be reduced.

A contact state between the ventilation adapter 7 and the rubber packing 44 will be described. Although the humidified CO₂ gas is held in the gas space portion 8 of the ventilation adapter 7, a structure having high airtightness is desirable in order to reduce leakage of the humidified CO₂ gas from a gap between the ventilation adapter 7 and the rubber packing 44.

The humidified CO₂ gas is continuously supplied to the internal space of the ventilation adapter 7, so that the internal space of the ventilation adapter 7 is maintained at a pressure slightly higher than the atmospheric pressure. That is, the internal space of the ventilation adapter 7 has a positive pressure compared with the atmospheric pressure. Accordingly, the risk that bacteria in the atmosphere enter the internal space of the ventilation adapter 7 is reduced.

A volume of the internal space of the ventilation adapter 7 is preferably as small as possible as long as the humidified CO₂ gas can be uniformly supplied to the gas permeable membrane of the culture vessel. This is because when the volume of the internal space is large, the time for filling the internal space with the humidified CO₂ gas becomes long.

On the other hand, when the internal space is too small, the humidified CO₂ gas may not be supplied uniformly. Therefore, it is preferable that the internal space is a space that is as thin as possible in an up-down direction and has a shape having an area equal to or slightly larger than an area of the gas permeable membrane of the culture vessel in a horizontal direction. The space shape is circular when the vessel is cylindrical, and is square when the vessel is rectangular.

FIG. 3B illustrates a ventilation adapter in which CO₂ gas is supplied from the bottom surface and exhausted from the bottom surface, FIG. 3B illustrates a state before the culture vessel 1, the rubber packing 44, and the ventilation adapter 7 are placed, and FIG. 3C illustrates a state during use. The ventilation adapter of this type is characterized in that the internal space of the ventilation adapter 7 can be smaller than that of the type illustrated in FIG. 3A. Further, in FIG. 3B, the cross-sectional shape of the rubber packing 44 is a shape extending along the outer periphery of the culture vessel in order to enhance the airtightness between the rubber packing 44 and the culture vessel. Even if the inner circumferential circle of the shape is smaller than the outer circumferential edge of the culture vessel, the rubber packing 44 is closely attached to the ventilation adapter 7 by utilizing the elasticity of the rubber packing.

The form of the ventilation adapter can be appropriately selected according to the required specifications (the size of the culture vessel, the culture speed, and the like) of the culture device. In addition to the forms illustrated in FIGS. 3A and 3B, it is also possible to supply a CO₂ gas supply pipe from the outside of the circumference and provide an exhaust pipe on the bottom surface.

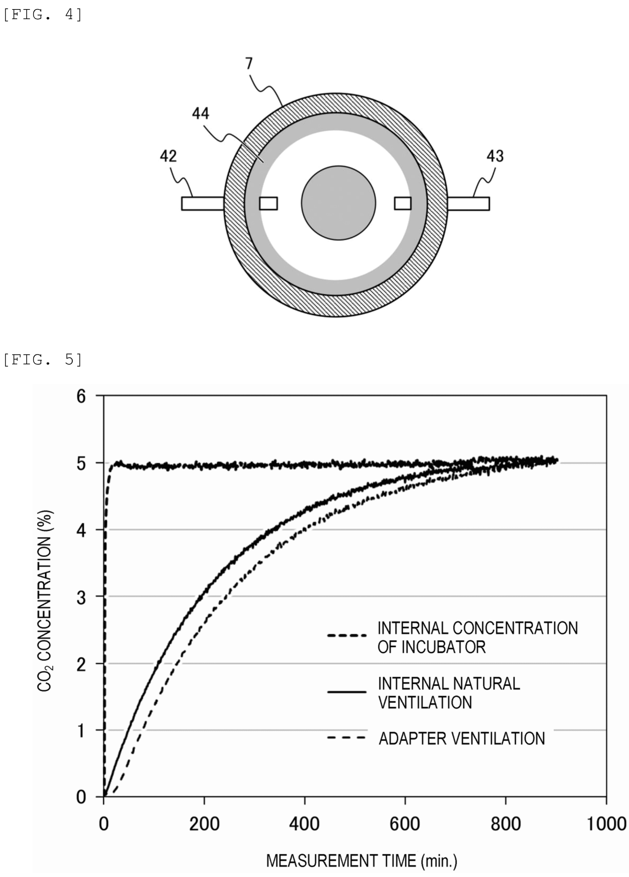

FIG. 4 is a top view of the ventilation adapter 7 in the form shown in FIG. 3A as viewed from above. As described above, the internal space of the ventilation adapter 7 has a circular shape slightly larger than the circular gas permeable membrane provided in the culture vessel when viewed from above. In this drawing, there is one gas feeding pipe 42 for the humidified CO₂ gas. Alternatively, the gas feeding pipe may be divided into a plurality of pipes so that the humidified gas CO₂ can be supplied to the internal space as uniformly as possible, or the opening may be one large opening. The gas feeding pipe 42 is not limited to the connection of the side surface of the ventilation adapter 7, and may be connected from a lower surface direction.

FIG. 5 illustrates the results of comparing the temporal changes in concentrations of the CO₂ gas as a gas phase in the culture vessel in Embodiment 1 using two measurement methods, a ventilation method using the ventilation adapter 7 and a ventilation method using a 5% CO₂ incubator.

For the measurement based on the ventilation method using the ventilation adapter 7, a CO₂ sensor (VISSLA GM70 handy type CO₂ meter) (not shown) was placed inside the closed culture vessel 1, the vent filter 6 was temporarily closed, and a vessel having only the ventilation surface (gas permeable membrane) 4 for gas passage was produced. The volume of the gas space portion 8 was 300 cc, and a mixed gas of 5% CO₂-air was continuously fed at a flow rate of 50 cc/min from the gas feeding pipe 42.

In the measurement based on the ventilation method using the CO₂ incubator, a CO₂ sensor was placed inside the incubator always maintained with 5% CO₂-air, a separate CO₂ sensor was placed inside the closed culture vessel 1 simultaneously, and a vessel in which a gas passes only the ventilation surface (gas permeable membrane) 4 was produced. The two vessels were maintained at 37°C, and measurements were performed for 15 hours to obtain the measurement results.

As a result, the 5% CO₂ concentration was reached in 800 minutes in both the ventilation method using the CO₂ incubator and the ventilation method using the ventilation adapter.

In the ventilation using the ventilation adapter, the same ventilation as the ventilation using the CO₂ incubator can be performed by adjusting the gas feeding amount in consideration of the fact that it takes time to increase the gas concentration of the gas space portion 8.

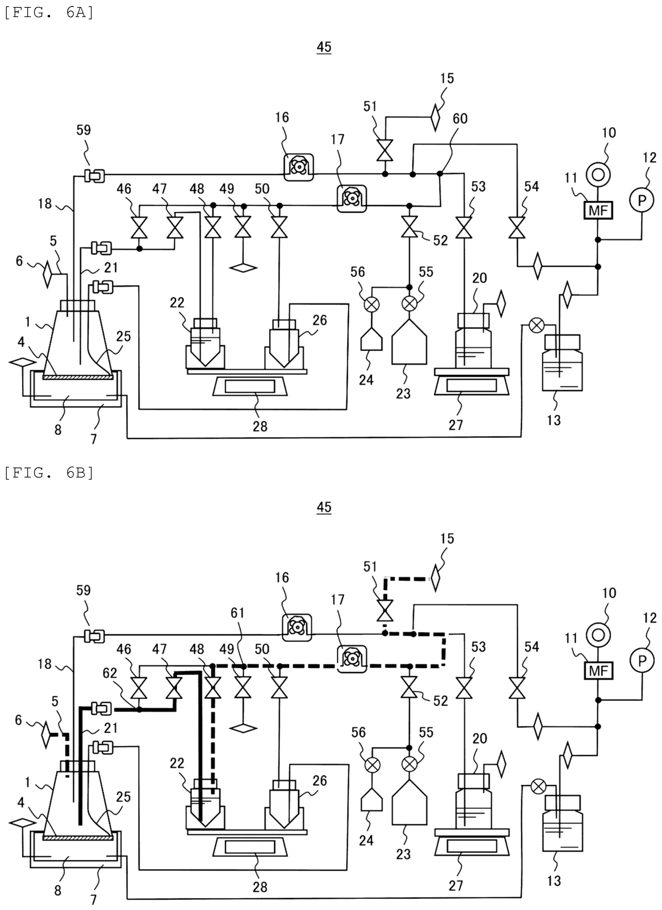

FIG. 6A is a view illustrating an example of a flow path circuit 45 including a closed culture vessel according to Embodiment 1. The same elements as those in the embodiment shown in FIG. 1 are denoted by the same reference signs, and the specific solenoid valves 19-1 to 19-9 for the solenoid valve 19 are denoted by the reference signs 46 to 54, respectively.

The rubber tube connected to the supernatant collection bag 23 is provided with a manual valve 55 by which the tube can be manually opened and closed, and the rubber tube connected to the supernatant analysis bag 24 is provided with a manual valve 56. The reference sign 59 denotes a joint component, which is a component by which the joining and disconnection of pipelines are enabled by using male and female joints. The reference sign 60 denotes a branching portion, which is a branching point of tubes joined using a T-shaped connection component.

The flow path circuit 45 shown here can be integrally attached to and detached from the device body shown in Embodiment 1 except for the solenoid valve 19, the weight sensor (electronic balance) 28, the gas flow rate control unit 11, and the pressure sensor 12 in the pumps 16 and 17 as mechanical elements, and can be applied to cell culture by sterilizing only the flow path circuit.

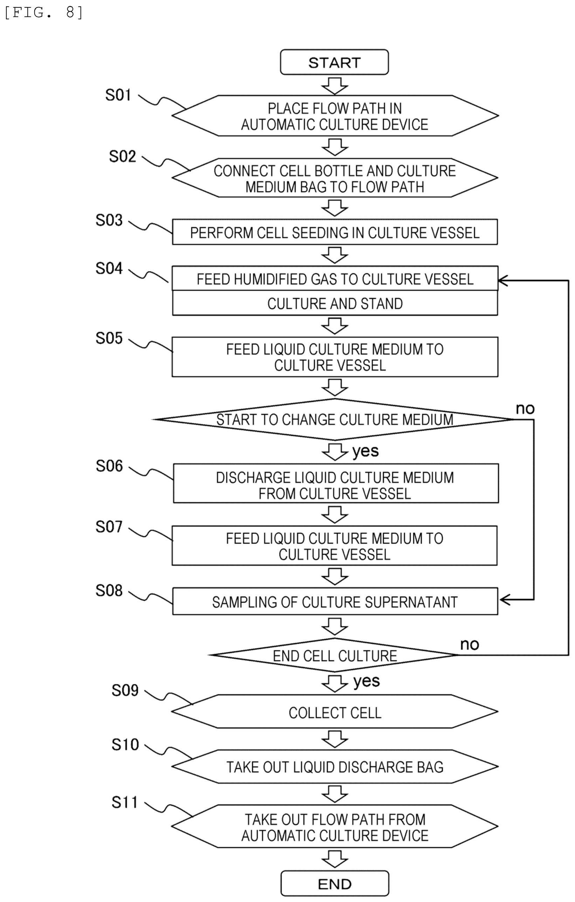

FIG. 8 is a flowchart of an overall operation of cell culture in the cell culture device 100 illustrated in FIG. 6A. After "START", the flow path is placed in the cell culture device 100 (S01). Then, the cell seeding bottle 22 for holding the separately prepared cell suspension, the culture medium bottle 20 for holding the culture medium, and the cell collection bottle 26 are connected to the flow path (S02).

FIG. 6B illustrates a step of cell seeding according to Embodiment 1. In an initial state, since the pumps are stopped and the roller is stopped by clamping the rubber tube, the pumps 16 and 17 are closed as valves. The solenoid valve is closed by clamping the rubber tube. A predetermined amount of a cell suspension is held in the cell seeding bottle 22, and the cell seeding bottle 22 is placed in the weight sensor 28. The culture vessel 1 is horizontally placed on the ventilation adapter 7 in an empty state. At the start of cell seeding, the solenoid valve 47, the solenoid valve 48, the solenoid valve 54, and the solenoid valve 51 are opened, and a pipeline from the aspirating tube 21 of the culture vessel 1 to the CO₂ gas vent filter 15 is opened.

Next, when the pump 17 is operated and a gas is fed from one of pipelines of the cell seeding bottle 22 to apply pressure to the cell suspension inside the bottle, the cell suspension passes through a pipeline and is fed from the aspirating tube 21 to the culture vessel 1 (S03). A flow state of the liquid at this time is indicated by a solid line, and a flow state of the gas is indicated by a broken line (the same applies hereinafter).

Next, when a predetermined amount of the cell suspension has been moved, the solenoid valve 47 is closed, the solenoid valve 49 is opened, and the pump 17 is stopped simultaneously. At this time, the cell suspension is temporarily stopped in the pipeline by closing the solenoid valve 47, the gas pressurizing the cell seeding bottle 22 is discharged to the vent filter opened from a branching portion 61 to the outside, and the movement of the liquid is stopped.

Next, when the solenoid valves 48 and 49 are closed, the solenoid valve 46 is opened, and the pump 17 is operated, the cell suspension in the pipeline closer to the culture vessel than a branching portion 62 is fed to the culture vessel 1. Thereafter, when the pump 17 is stopped and the solenoid valves 47, 48, and 49 are opened, the cell suspension in the pipeline returns to the cell seeding bottle 22 due to the difference in height, there is no liquid in the pipeline, and all the solenoid valves are closed to end the cell seeding step (S03).

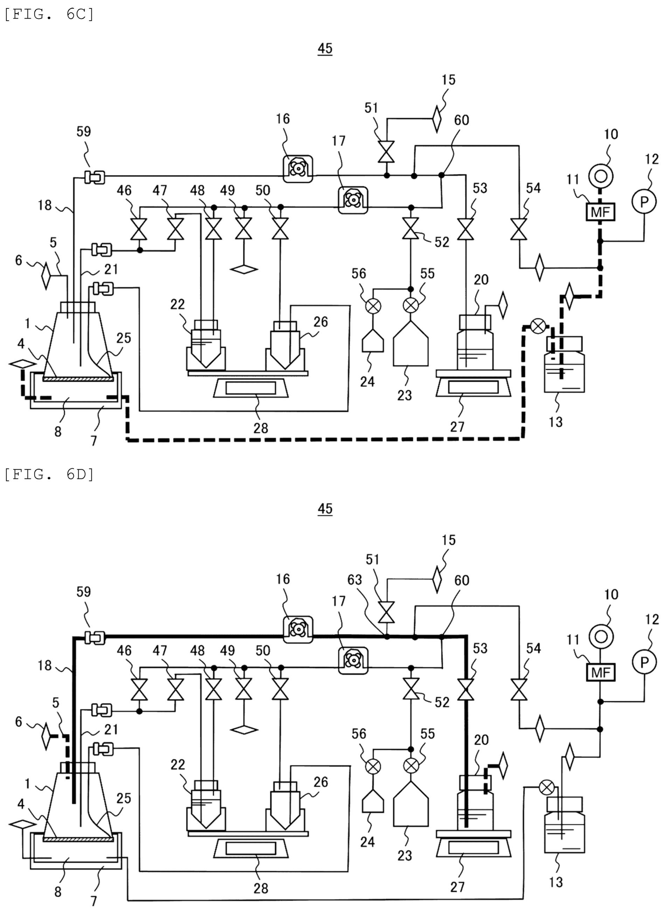

FIG. 6C illustrates a step of exchanging gas in the culture vessel 1 in Embodiment 1. In the initial state, the solenoid valve is closed by closing the rubber tube. Water is held in the humidification bottle 13, and an opening of one long pipe is provided at a bottom portion of the vessel. The gas flow rate control unit 11 and the gas cylinder 10 are connected to one end of the long pipe.

An opening of a short tube in the humidification bottle 13 is provided at an upper portion of the vessel, and the ventilation adapter is connected to one end of the short tube. When the gas flow rate control unit 11 is operated, the gas controlled at a predetermined air feeding amount is fed into the humidification bottle, humidified, and fed from the humidification bottle. Next, a gas concentration of the gas space portion 8 of the ventilation adapter 7 is increased and maintained at a predetermined gas concentration, and therefore, the gas exchange in the culture vessel 1 is continuously performed (S04).

FIG. 6D illustrates the step of adding the culture medium to the culture vessel 1 in Embodiment 1. In an initial state, the pump is stopped, and the solenoid valve is closed by closing the rubber tube. The culture medium is held in the culture medium bottle 20, and the culture medium bottle 20 is placed on the weight sensor 27. First, the solenoid valve 53 is opened to open a pipeline from the liquid feeding pipe 18 of the culture vessel 1 to the culture medium bottle 20. Next, when the pump 16 is operated, the culture medium passes through the pipeline and is fed from the liquid feeding pipe 18 to the culture vessel 1.

Next, when a predetermined liquid amount of the culture medium has been moved, the solenoid valve 51 is opened, and the pump 16 is stopped simultaneously. At this time, the flow of the culture medium is temporarily stopped in the pipeline by the stop of the pump 16, and the culture medium in the pipeline close to the culture medium bottle 20 returns to the inside of the culture medium bottle 20 due to the difference in height after the outside air enters from the vent filter opened from a branching portion 63 to the outside.

Next, when the solenoid valve 53 is closed and the pump 16 is operated, the culture medium in the pipeline closer to the culture vessel than the branching portion 63 is fed to the culture vessel 1. Thereafter, when the pump 16 is stopped, there can be no liquid in the pipeline, and all the solenoid valves are closed to end the culture medium addition step (S05).

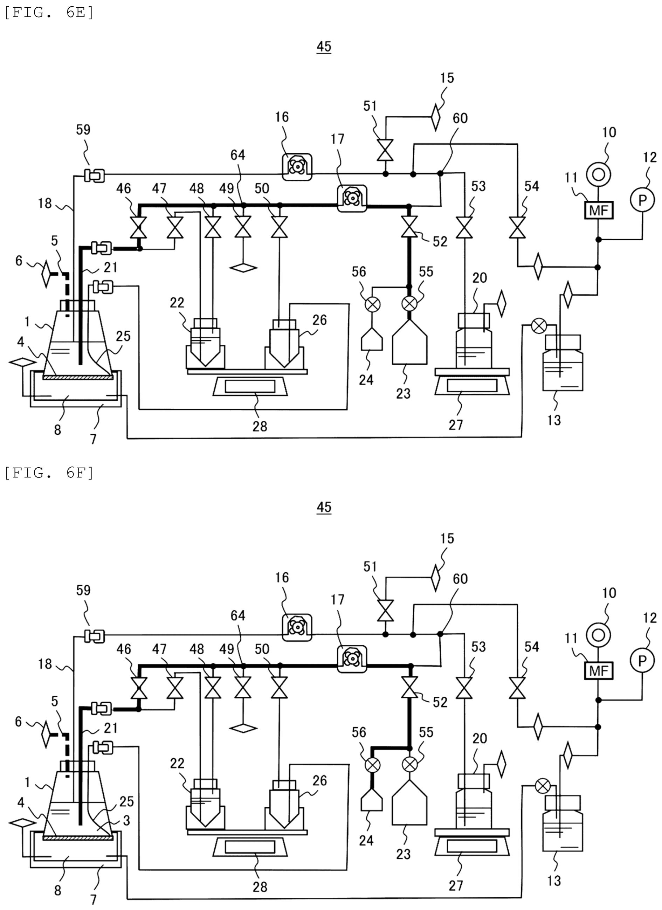

FIG. 6E illustrates a step of discharging the culture medium in culture medium change in the culture vessel 1 in Embodiment 1. In an initial state, the pump is stopped, and the solenoid valve is closed by closing the rubber tube. The culture medium is held in the culture vessel 1, and the supernatant collection bag 23 is empty. First, the solenoid valve 46, the solenoid valve 52, and the manual valve 55 are opened, and a pipeline from the aspirating tube 21 of the culture vessel 1 to the supernatant collection bag 23 is opened. Next, when the pump 17 is operated, the culture medium is fed from the culture vessel 1 through the aspirating tube 21 and reaches the supernatant collection bag 23.

Next, when the solenoid valve 49 is opened, the culture medium in the pipeline closer to the culture vessel 1 than a branching portion 64 (the same as the branching portion 61 in FIG. 6B) is fed to the culture vessel 1 by the difference in height. Thereafter, when the solenoid valve 46 is closed and the pump 17 is operated, the outside air is introduced from the vent filter, and the culture medium in the pipeline reaches the supernatant collection bag 23. All the solenoid valves are closed, and the culture medium discharge step ends (S06). Next, by performing the step of adding the culture medium to the culture vessel 1 described with reference to FIG. 6D in the same manner, the culture medium change can be performed (S07). After the culture medium is changed, the culture vessel 1 is swung by the swing mechanism 30 to mix a new culture medium with cells, thereby further promoting the cell culture.

FIG. 6F illustrates a step of sampling the supernatant from the culture vessel 1 in Embodiment 1. In an initial state, the pump is stopped, and the solenoid valve is closed by closing the rubber tube. The culture medium is held in the culture vessel 1, and the supernatant analysis bag 24 is empty. First, the solenoid valve 46, the solenoid valve 52, and the manual valve 56 are opened, and a pipeline from the aspirating tube 21 of the culture vessel 1 to the supernatant analysis bag 24 is opened. Next, when the pump 17 is operated, the culture medium is fed from the culture vessel 1 through the aspirating tube 21 and reaches the supernatant analysis bag 24.

Next, when the solenoid valve 49 is opened, the culture medium in the pipeline closer to the culture vessel 1 than the branching portion 64 is fed to the culture vessel 1 by the difference in height. Thereafter, when the solenoid valve 46 is closed and the pump 17 is operated, the outside air is introduced from the vent filter, and the culture medium in the pipeline reaches the supernatant analysis bag 24. All the solenoid valves are closed, and the supernatant sampling step ends (S08).

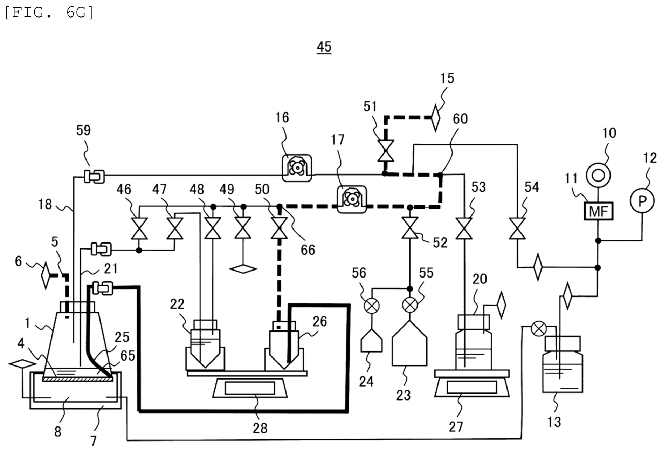

A step of collecting cells from the culture vessel 1 in Embodiment 1 will be described with reference to FIGS. 6E and 6G. After the cells have sufficiently grown, the cells settle to the bottom surface of the culture vessel and float thereon. Similarly to the supernatant collection step according to FIG. 6E, the supernatant is collected from the culture vessel 1 in the same flow path circuit, and the supernatant collection is continued until the culture medium 3 is below the height of an opening of the aspirating tube 21. Accordingly, an abundance ratio of the cells to the culture medium 3 in the culture vessel 1 can be increased. Next, the culture vessel is swung by the above-described swing mechanism 30, and the culture medium and the cells are stirred to obtain a cell suspension.

Next, the step of collecting cells from the culture vessel 1 will be described with reference to FIGS. 6G and 6A. In an initial state, the pump is stopped, and the solenoid valve is closed by closing the rubber tube. A cell suspension 65 is held in the culture vessel 1, and the cell collection bottle 26 is empty.

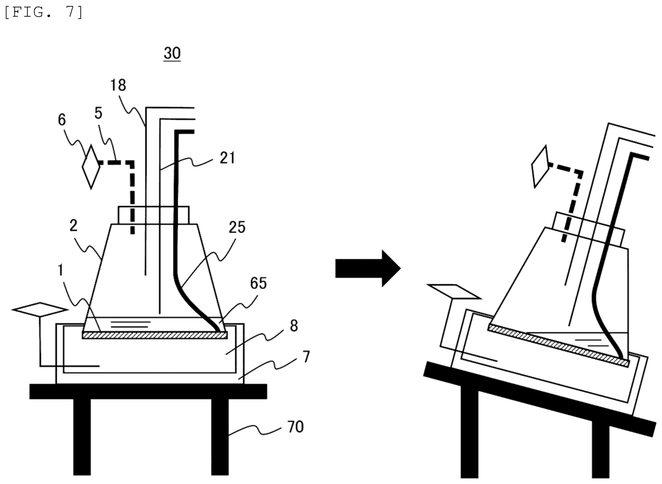

A state of inclination of the culture vessel during cell collection will be described with reference to FIG. 7. First, a swing unit 70 of the swing mechanism 30 is inclined in the direction in which the collection pipe 25 is present, so that the cell suspension is collected on a wall surface side of the culture vessel 1 in which the collection pipe 25 is present. An inclination angle of the swing unit 70 may be any angle as long as the cell suspension can be collected on the wall surface side of the culture vessel 1, and may be any angle larger than 0 degree and smaller than 90 degrees. The swing unit 70 needs to be capable of being inclined in a certain direction, and may be inclined in a plurality of directions. When the culture vessel 1 is placed on the swing mechanism, the culture vessel 1 is placed in a direction in which the culture vessel 1 can be inclined toward the direction in which the opening of the collection pipe 25 is present. At this time, a mark for designating the placement direction of the culture vessel 1 may be described on the ventilation adapter 7 fixed to the swing unit 70.

Next, the solenoid valves 50 and 51 are opened, and a pipeline from the collection pipe 25 of the culture vessel 1 to the cell collection bottle 26 and a pipeline to the CO₂ gas vent filter 15 opened to the outside air are opened.

Next, when the pump 17 is operated, the cell suspension 65 is fed from the culture vessel 1 through the collection pipe 25 and reaches the cell collection bottle 26.

Next, when the total amount of the cell suspension 65 has been moved to the cell collection bottle 26, the solenoid valve 49 is opened, and the pump 17 is stopped simultaneously. At this time, the outside air enters from the vent filter, which is opened to the outside, through a branching portion 66, the pressure inside the cell collection bottle 26 becomes a constant pressure, and the liquid feeding stops. All the solenoid valves are closed, and the cell collection step ends (S09).

After collecting the cells, a waste liquid bag is taken out from the flow path (S10), the flow path is taken out from the automatic culture device (S11), and all the steps of the automatic culture are completed.

As described above, the automatic culture device according to the present embodiment can perform culture while being reduced in the size thereof. It is possible to reduce a risk of occurrence of rust on mechanical elements such as a swing mechanism of a culture vessel used for a culture operation and a camera observation mechanism for cells, and to mount such mechanical elements. In addition, the risk of microorganism generation in the entire incubator can be reduced by the partial supply of the humidified gas to the culture vessel by the ventilation adapter.

### [Embodiment 2]

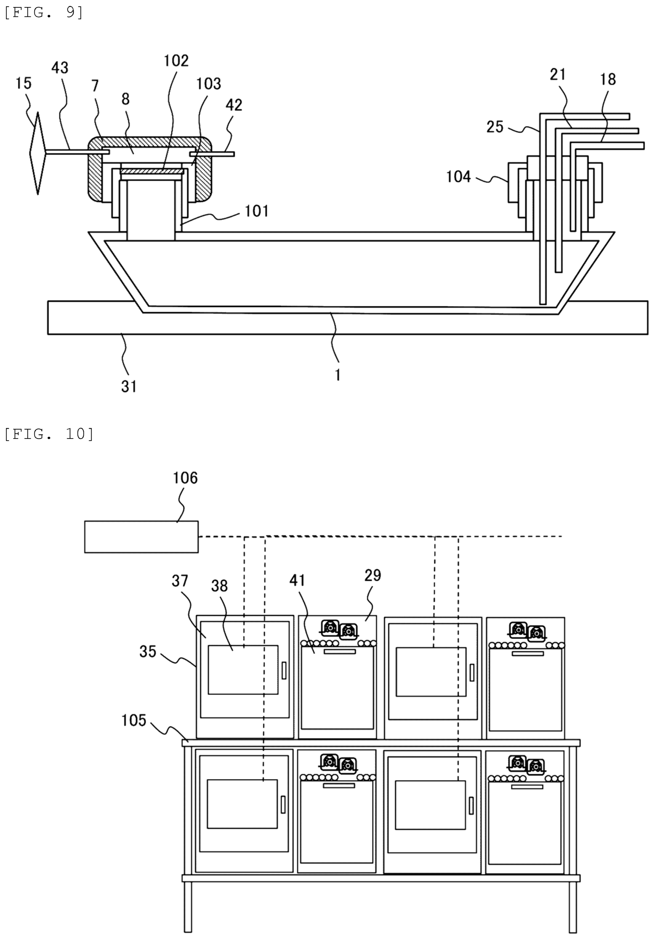

FIG. 9 is a diagram illustrating an example of the ventilation adapter for a flask culture vessel according to Embodiment 2.

Components of the ventilation adapter 7 according to Embodiment 2 will be described with reference to FIG. 9. The culture vessel 1 is a flask-type culture vessel, and is mainly used for cell culture with cells adhering to the bottom surface of the vessel. Normally, two screw ports 101 are provided on a ceiling surface on the main body side, and each screw port is closed by a ventilation cap 103 provided with a gas permeable film 102 at the opening.

In the present embodiment, a method of performing gas exchange by the ventilation adapter 7 with the ventilation cap of one screw port as it is is shown, and a ported cap 104 provided with a liquid feeding pipe is placed on the other screw port to perform automatic liquid feeding by the automatic culture device. More specifically, the culture vessel 1 is placed on the swing stage 31 and is connected to the gas feeding pipe 42 connected to the humidification bottle 13 and the exhaust pipe 43 connected to the CO₂ sensor. One ventilation cap 103 on the culture vessel 1 is in close contact with the ventilation adapter 7. At this time, the predetermined gas space portion 8 is formed inside the ventilation cap 103 and the ventilation adapter 7.

The ported cap 104 is provided on the liquid feeding pipe 18, the aspirating tube 21, and the cell collection pipe 25, and these three ports are connected in the same manner as the automatic culture device (FIG. 1) and the flow path circuit (FIG. 6A) described in Embodiment 1, and the cell seeding step, the gas exchange step, the culture medium addition step, the culture medium change step, the supernatant sampling step, and the cell collection step can be performed.

### [Embodiment 3]

FIG. 10 is a diagram illustrating an example of an automatic culture system in which a plurality of automatic culture devices according to Embodiment 1 are placed, connected to a network, and controlled by a central management PC.

Four sets of automatic culture devices, each of which is a set of the incubator 35 and the fluid control unit 29, are disposed on a rack 105. In this embodiment, a rack on which four sets of devices are disposed, two sets on each of upper and lower stages, is shown. However, the number of placements per stage of the rack of this embodiment is any number, and it is easy to increase the number of integrated devices in a limited facility space of the CPC. From the viewpoint of accurate process management and sterile operation, it is also possible to fully automate all of the placement and removal processes of the flow path by a robot, and it is possible to manufacture the flow path beyond a working range that can be reached by a human hand.

Each control unit 38 is attached to a front surface of the door 37 of the incubator 35 and is connected to a central control PC 106 by network equipment. The central control PC 106 has a function of monitoring a state of each of the automatic culture devices, a function of managing update and correction of an operation program, and a function of transmitting information when an operation abnormality of the device occurs or the operation ends.

By adopting the configuration described in the present embodiment, it is possible to provide an automatic culture device in which a plurality of devices can be operated simultaneously in a space-saving manner, and an automatic culture system using a plurality of automatic culture devices.

### Reference Signs List

- 1:: culture vessel
- 2:: cell
- 3:: culture medium
- 4:: ventilation surface
- 5:: pressure regulating pipe
- 6:: vent filter
- 7:: ventilation adapter
- 8:: gas space portion
- 9:: gas supply unit
- 10:: gas cylinder
- 11:: flow rate control unit
- 12:: pressure sensor
- 13:: humidification bottle
- 14:: CO₂ sensor
- 15:: CO₂ gas vent filter
- 16, 17:: pump
- 18:: liquid feeding pipe
- 19:: solenoid valve
- 20:: culture medium bottle
- 21:: aspirating tube
- 22:: cell seeding bottle
- 23:: supernatant collection bag
- 24:: supernatant analysis bag
- 25:: cell collection pipe
- 26:: cell collection bottle
- 28:: weight sensor
- 29:: fluid control unit
- 30:: swing mechanism

## Claims

1. An automatic culture device capable of accommodating a culture vessel, comprising:
a temperature maintaining mechanism configured to maintain the culture vessel at a predetermined temperature;
a gas supply unit configured to supply humidified CO₂ gas; and
a ventilation adapter attached to the culture vessel and configured to supply the humidified CO₂ gas from the gas supply unit to the culture vessel.

2. The automatic culture device according to claim 1, wherein
the culture vessel includes a gas permeable portion, at least a part of a vessel accommodating a culture target being made of a material having gas permeability, and
the ventilation adapter supplies the humidified CO₂ gas from the gas supply unit to the gas permeable portion of the culture vessel.

3. The automatic culture device according to claim 1, wherein
the ventilation adapter is configured separately from the culture vessel and is detachable from the culture vessel.

4. The automatic culture device according to claim 3, wherein
the ventilation adapter is a structure that the culture vessel is placed above the ventilation adapter and is attached to the ventilation adapter by being pushed down.

5. The automatic culture device according to claim 3, wherein
the ventilation adapter is a structure attached to the culture vessel by being pushed down from above the culture vessel toward the culture vessel.

6. The automatic culture device according to claim 1, wherein
a flow path for supplying a gas from the gas supply unit into the ventilation adapter and a flow path for discharging a gas in the ventilation adapter are connected to a side wall surface of the ventilation adapter.

7. The automatic culture device according to claim 1, wherein
a flow path for supplying a gas from the gas supply unit into the ventilation adapter and a flow path for discharging a gas in the ventilation adapter are connected to a bottom surface of the ventilation adapter.

8. The automatic culture device according to claim 1, wherein
the temperature maintaining mechanism includes a housing configured to house at least the ventilation adapter and the culture vessel, and a temperature adjustment mechanism configured to maintain a temperature in the housing at the predetermined temperature.

9. The automatic culture device according to claim 8, wherein
a flow path for discharging a gas in the ventilation adapter is connected to the ventilation adapter, and the gas is discharged outside of the housing.

10. The automatic culture device according to claim 9, wherein
a gas sensor configured to measure an amount of the gas discharged from the ventilation adapter is provided in the flow path for discharging the gas.

11. The automatic culture device according to claim 8, wherein
a swing mechanism configured to swing the culture vessel is provided inside the housing.

12. The automatic culture device according to claim 8, wherein
a humidifying unit configured to humidify the fed gas is provided inside the housing as a part of the gas supply unit.

13. The automatic culture device according to claim 8, comprising:
a fluid control unit outside the housing of the temperature maintaining mechanism, the fluid control unit including a first vessel configured to be capable of accommodating a supply culture medium and the like, a second container configured to be capable of accommodating a waste liquid and the like, and a pump that enables a fluid operation.

14. An automatic culture system comprising:
a plurality of the automatic culture devices according to any one of claims 1 to 13; and
a centralized management computer configured to control the plurality of automatic culture devices.

15. The automatic culture system according to claim 14, wherein
the plurality of automatic culture devices are arranged vertically or horizontally in parallel.
